(19) Europäisches Patentamt
European Patent Office
Office européen des brevets

(11) Veröffentlichungsnummer: **0 271 591**
**B1**

# (12) EUROPÄISCHE PATENTSCHRIFT

(45) Veröffentlichungstag der Patentschrift: **20.09.89**

(51) Int. Cl.⁴: **A61K 31/50**
// (A61K31/50, 31:275)

(21) Anmeldenummer: **86117382.1**

(22) Anmeldetag: **13.12.86**

(54) **Kombinationspräparat.**

(43) Veröffentlichungstag der Anmeldung:
**22.06.88 Patentblatt 88/25**

(45) Bekanntmachung des Hinweises auf die Patenterteilung:
**20.09.89 Patentblatt 89/38**

(84) Benannte Vertragsstaaten:
**AT BE CH ES FR GB IT LI NL SE**

(56) Entgegenhaltungen:
**EP-A- 0 064 158**
**EP-A- 0 117 403**

(73) Patentinhaber: **BASF Aktiengesellschaft,**
**Carl-Bosch-Strasse 38, D-6700 Ludwigshafen(DE)**

(72) Erfinder: **König, Horst, Prof. Dr., Pariser Strasse 4,**
**D-6700 Ludwigshafen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein neues Kombinationspräparat zur Behandlung von Durchblutungsstörungen auf thromboembolischer Grundlage.

Es ist bekannt, daß mit basisch substituierten Phenylacetonitrilen (vgl. DE-PS 1.154.810, EP-OS 64.158) Herzkrankheiten, die durch eine mangelhafte Gewebsdurchblutung (Ischämie) verursacht sind, behandelt werden können (vgl. die Liste Pharmindex III/84, Isoptin ® und Procorum ®). Die Wirkung beruht sowohl auf einer gefäßdilatierenden als auch auf einer kardioprotektiven und einer thrombocytenaggregationshemmenden Wirkung. Es ist weiter bekannt (vgl. DE-OS 2.845.220, DE-OS 3.124.699, EP-OS 117.403), daß bestimmte Pyridazinon-Derivate eine thrombocytenaggregationshemmende Wirkung besitzen.

Es wurde nun gefunden, daß sich die Wirkung der Phenylacetonitrile durch Pyridazinon-Derivate stark erhöhen läßt.

Gegenstand der Erfindung ist ein Arneimittel, dadurch gekennzeichnet, daß es eine Verbindung der Formel I

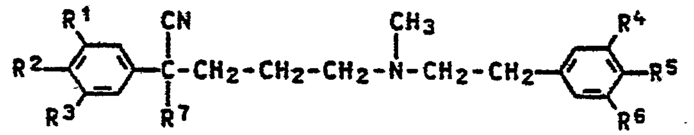

I,

worin

$R^1$-$R^6$ gleich oder verschieden sind und Wasserstoffatome, Halogenatome, $C_{1-4}$-Alkylgruppen oder $C_{1-4}$-Alkoxygruppen bedeuten und $R^7$ einen gesättigten oder ungesättigten Kohlenwasserstoffrest mit bis zu 20 Kohlenstoffatomen

bedeutet, und ein Pyridazinon-Derivat der Formel II

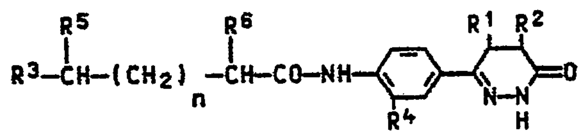

II,

worin

$R^1$ ein Wasserstoffatom oder eine Methylgruppe ist,

$R^2$ ein Wasserstoffatom oder zusammen mit $R^1$ eine Methylen- oder Ethylengruppe darstellt,

$R^3$

a) eine Gruppe der Formel

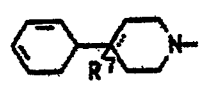

worin die gestrichelte Linie eine zusätzliche Bindung bedeuten kann und $R^7$ gegebenenfalls ein Wasserstoffatom oder einen $C_{1-4}$-Acylrest darstellt, oder

b) eine Gruppe der Formel

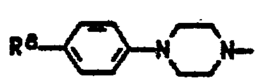

worin $R^8$ ein Wasserstoffatom oder eine $C_{1-4}$-Acylgruppe darstellt, oder

c) den 1,3-Tetrahydroisochinolin-2-yl-Rest bedeutet,

$R^4$ ein Wasserstoffatom oder zusammen mit $R^3$ eine direkte Bindung oder eine Methylengruppe darstellt,

$R^5$ ein Wasserstoffatom oder eine Methylgruppe ist,

$R^6$ ein Wasserstoffatom oder ein Halogenatom bedeutet und

n die Zahl 0 oder 1 ist,

in einer Menge von 50-450 Gewichtsteilen der Verbindung I und 3-50 Gewichtsteilen der Verbindung II enthält.

In dem Arzneimittel können die Verbindungen der Formel I in freier Form oder in Form ihrer physiologisch verträglichen Salze vorliegen. Zur Salzbildung mit den Verbindungen I eignen sich Salzsäure, Schwefelsäure, Phosphorsäure, Essigsäure, Malonsäure, Bernsteinsäure, Fumarsäure, Maleinsäure,

Zitronensäure, Weinsäure, Milchsäure, Amidosulfonsäure und Oxalsäure. Vorzugsweise werden die Verbindungen der Formel I in Form ihrer Hydrochloride eingesetzt.

Das angegebene Mischungsverhältnis bezieht sich auf die freie Form der Verbindungen I. Der bevorzugte Bereich des Mischungsverhältnisses liegt bei etwa 100 Teilen Verbindung I und 10 Teilen Verbindung II.

Die überlegene Wirkung der neuen Kombination läßt sich durch Bestimmung der Thrombocytenaggregation in folgenden Versuchen zeigen:

1. Thrombozytenreiches Plasma wird durch Zentrifugation (300 g, 10 min Dauer bei 4°C) von venösem Citratblut gewonnen. Die photometrische Messung der Thrombozytenaggregation erfolgt unter Zusatz von $MgCl_2$ (Endkonzentration 10 mmol/l) und von Collagen Stago (Endkonzentration 0,02 mg/ml) im Born-Aggregometer Mk 3. Als Aggregationsmaß findet die maximale Extinktionsänderung/sec Verwendung.
Die Prüfung der aggregationshemmenden Wirksamkeit der Substanzen wird nach einer Inkubationszeit von 10 min vorgenommen.
Als EC 50 % wird die Konzentration bestimmt, welche eine 50 %ige Hemmung der Aggregation verursacht.

2. Beagle-Hunde (10 bis 15 kg Körpergewicht) erhalten die Substanzen bzw. die Kombination oral. Vor sowie 2 und 4 h nach der Substanzabgabe wird venöses Blut entnommen, dieses durch Citrat-Zusatz ungerinnbar gemacht und daraus durch anschließende Zentrifugation (3cc g, 1c min. Dauer bei 4°C) thrombozytenreiches Plasma gewonnen. Zugabe von Adrenalin (Endkonzentration $5 \times 10^{-8}$ mol/l) und Kollagen (Endkonzentration $2 \times 10^{-3}$ g/l) zu thrombozytenreichem Plasma löst eine Aggregation aus, die als Extinktionslösung im Born-Aggregometer MK 3 gemessen wurde. Als Aggregationsmaß findet die maximale Extinktionsänderung pro Sekunde Verwendung. Aus dem Vergleich der Werte vor und nach Gabe der Substanz wurde die prozentuale Hemmung der Aggregation ermittelt.

In den Versuchen wurden folgende Ergebnisse erhalten:

| Versuch | Substanz | Dosis (mg/kg) | Aggregationshemmung (%) |
|---|---|---|---|
| 2 | Amipizone* | 0,1 | 2,0 |
| 2 | Verapamil | 0,1 | 2,8 |
| 2 | Amipizone + Verapamil | 0,1 + 0,1 | 18 |
| 1 | Amipizone | 0,005 | 23 |
| 1 | Amipizone | 0,1 | 48 |
| 1 | Verapamil | 21,5 | 17 |
| 1 | Amipizone + Verapamil | 0,005 + 21,5 | 50 |
| 1 | Amipizone + Verapamil | 0,01 + 21,5 | 77 |

* Amipizonne vgl. EP-OS 117 403, Beispiel 13

Die neue Kombination eignet sich zur Behandlung und Prophylaxe von Erkrankungen, die durch Thrombocytenaggregation ausgelöst werden. Dazu gehören thromboembolische Erkrankungen im Bereich von Herz, Gehirn und peripherem, arteriellem Gefäßsystem. Solche Erkrankungen sind beispielsweise Herzinfarkt, Schlaganfall sowie obliterierende Arteriosklerose.

Die neue Kombination ist weiterhin geeignet zur Behandlung und Prophylaxe von Erkrankungen, die durch die Freisetzung vasoaktiver Stoffe aus aggregierenden Thrombocyten hervorgerufen werden, wie Migräne, vasospastische Angina und Morbus Raynaud. Sie ist auch nützlich zur Verhütung von Komplikationen bei chirurgischen Maßnahmen, wie Gefäßprothesen und Shunts.

Die neue Kombination zeigt bei der Anwendung auch weniger Nebenwirkungen als nach Gabe der Einzelbestandteile zu erwarten wären.

Die erfindungsgemäße Kombination kann in üblicher Weise oral verabfolgt werden.

Die Dosierung hängt vom Alter, Zustand und Gewicht des Patienten sowie von der Applikationsart ab. In der Regel beträgt die tägliche Dosis zwischen etwa 50 und 500 mg, vorzugsweise 50 und 200 mg, Verbindung I und zwischen etwa 3 und 50, vorzugsweise etwa 10 mg, Verbindung II pro Patient und Tag.

Die neue Kombination kann in den gebräuchlichen galenischen Applikationsformen fest oder flüssig angewendet werden, z.B. als Tabletten, Filmtabletten, Kapseln, Pulver, Granulate, Dragees, Pellets, Retardpellets oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 99 Gewichtsprozent.

Beispiel 1

Auf einer Tablettenpresse werden in üblicher Weise Tabletten folgender Zusammensetzung gepreßt:

400 mg Verapamilhydrochlorid
10 mg 2-(1,2,3,4-Tetrahydrochinolin-2-on-6-yl)-3,4-diaza-bicyclo[4.1.0]-hepten-(2)-on(5)
72 mg Maisstärke
73 mg Gelatine
35 mg Milchzucker
25 mg Carboxymethylcellulose
17,5 mg Talcum
3,5 mg Magnesiumstearat

Beispiel 2

In üblicher Weise werden Drageekerne folgender Zusammensetzung hergestellt:

50 mg Gallopamil
5 mg 2-(2,3,4,5-Tetrahydro-benzo[b]azepin-2-(1H)-on-7-yl)-3,4-diazabicyclo[4.2.0]-octen-(2)-on-(5)
50 mg Kernmasse

Die Kernmasse besteht aus 9 Teilen Maisstärke, 3 Teilen Milchzucker und 1 Teil Luviskol] VA 64 (Vinylpyrrolidon-Vinylacetat-Mischpolymerisat 60 : 40, vgl. Pharm. Ind. 1962, 586).

Die erhaltenen Kerne werden anschließend mit einer Verzuckerungsmasse folgender Zusammensetzung überzogen:

5 Teile Saccharose
2 Teile Maisstärke
2 Teile Calciumcarbonat
1 Teil Kalk

Beispiel 3

Zur Herstellung von Filmtabletten werden die gemäß Beispiel 2 hergestellten Drageekerne mit einem Filmüberzug folgender Zusammensetzung überzogen:

Tylose 0,7%
®Kollidon 25 (PVP) 0,4%
Saccharose 70,0%
hochdisperse Kieselsäure 1,4%
Talcum 11,0%
Maisstärke 2,0%
Calciumcarbonat 3,5%
Gummi arabicum 2,5%
Titandioxid + Farbstoffe 8,5%

Beispiel 4

Es werden in Steckkapseln abfüllbare Retardpellets hergestellt, wobei jede Komponente für sich pelletiert und die Retardpellets anschließend gemischt oder nacheinander in die Kapseln gefüllt werden.

Zusammensetzung der Pellets pro Dosis.

Retardpellets, Verbindung I:

100 mg Gallopamilhydrochlorid
60 mg Cellulosepulver
5 mg Maisstärke
10 mg Talkum
35 mg Ethylcellulose

Retardpellets, Verbindung II:

20 mg 6-[p-3-(4-Phenyl-1-piperidyl)-propionylamino-phenyl]-4,5-dihydro-5-methyl-3(2H)-pyridazinon
20 mg Cellulosepulver
10 mg Maisstärke
10 mg Ethylcellulose

Beispiel 5

Es werden in Steckkapseln abfüllbare Pellets hergestellt mit folgender Zusammensetzung:

300 mg Verapamil
5 mg 6-[p-3-(4-Phenyl-1-piperazinyl)-propionylamino-phenyl]-4,5-dihydro-5-methyl-3(2H)-pyridazinon
80 mg Cellulosepulver
30 mg Maisstärke
5 mg ®Kollidon 30 (PVP) 20 mg ®Eudragit S (Polymerisat aus Methylacrylsäure und Methacrylsäureester
15 mg Talkum

**Patentansprüche**

1. Arzneimittel, dadurch gekennzeichnet, daß es eine Verbindung der Formel I

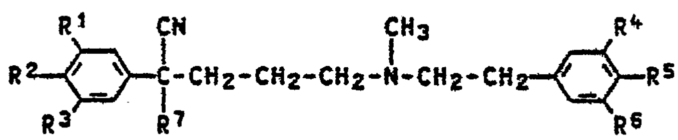 I,

worin

$R^1$-$R^6$ gleich oder verschieden sind und Wasserstoffatome, Halogenatome, $C_{1-4}$-Alkylgruppen oder $C_{1-4}$-Alkoxygruppen bedeuten und $R^7$ einen gesättigten oder ungesättigten Kohlenwasserstoffrest mit bis zu 20 Kohlenstoffatomen

bedeutet, und ein Pyridazinon-Derivat der Formel II

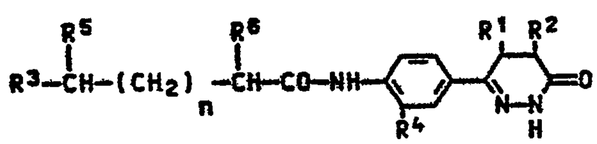 II,

worin
$R^1$ ein Wasserstoffatom oder eine Methylgruppe ist,
$R^2$ ein Wasserstoffatom oder zusammen mit $R^1$ eine Methylen- oder Ethylengruppe darstellt,
$R^3$
a) eine Gruppe der Formel

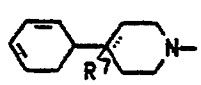

worin die gestrichelte Linie eine zusätzliche Bindung bedeuten kann und $R^7$ gegebenenfalls ein Wasserstoffatom oder einen $C_{1-4}$-Acylrest darstellt, oder

b) eine Gruppe der Formel

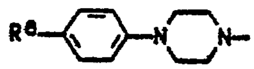

worin $R^8$ ein Wasserstoffatom oder eine $C_{1-4}$-Acylgruppe darstellt, oder

c) den 1,3-Tetrahydroisochinolin-2-yl-Rest bedeutet,

$R^4$ ein Wasserstoffatom oder zusammen mit $R^3$ eine direkte Bindung oder eine Methylengruppe darstellt,
$R^5$ ein Wasserstoffatom oder eine Methylgruppe ist,
$R^6$ ein Wasserstoffatom oder ein Halogenatom bedeutet und
n die Zahl 0 oder 1 ist,
in einer Menge von 50-450 Gewichtsteilen der Verbindung I und 3-50 Gewichtsteilen der Verbindung II enthält.

2. Verfahren zur Herstellung eines Arzneimittels gemäß Anspruch 1, dadurch gekennzeichnet, daß man 50-450 Gewichtsteile einer Verbindung der Formel I gemäß Anspruch 1 mit 3-50 Gewichtsteilen einer Verbindung der Formel II gemäß Anspruch 1 mischt und die so erhaltene Mischung zu einer galenischen Applikationsform verarbeitet.

**Revendications**

1. Médicament, caractérisé par le fait qu'il contient un composé de formule I

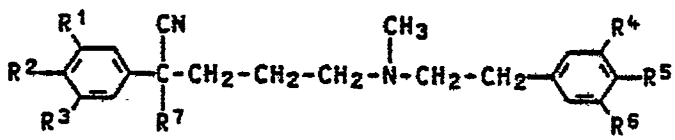 I,

dans laquelle
$R^1$-$R^6$, sont identiques ou différents et représentent des atomes d'hydrogène, atomes d'halogène, groupes alkyle en $C_{1-4}$ ou groupes alcoxy en $C_{1-4}$, et
$R^7$ représente un reste d'hydrocarbure saturé ou insaturé ayant 20 atomes de carbone,
et un dérivé de pyridazinone de formule II

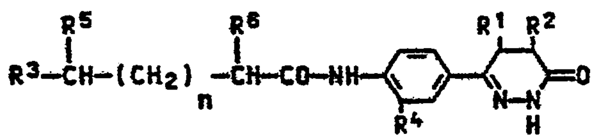 II,

dans laquelle
$R^1$ est un atome d'hydrogène ou un groupe méthyle,
$R^2$ représente un atome d'hydrogène ou, avec $R^1$, un groupe méthylène ou éthylène
$R^3$ représente

a) un groupe de formule

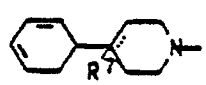

où la ligne en pointillé peut signifier une liaison supplémentaire et $R^7$ représente éventuellement un atome d'hydrogène ou un reste acyle en $C_{1-4}$, ou

b) un groupe de formule

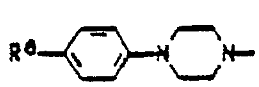

où $R^8$ représente un atome d'hydrogène ou un groupe acyle en $C_{1-4}$, ou

c) le reste 1,3-tétrahydroisoquinolin-2-yle

$R^4$ représente un atome d'hydrogène ou, avec $R^3$, une liaison directe ou un groupe méthylène
$R^5$ est un atome d'hydrogène ou un groupe méthyle
$R^6$ est un atome d'hydrogène ou un atome d'halogène, et
n est le nombre 0 ou 1
en quantités de 50–450 parties en poids du composé I et 3–50 parties en poids du composé II.

2. Procédé pour la préparation d'un médicament selon la revendication 1, caractérisé par le fait que l'on mélange 50–450 parties en poids d'un composé de formule I selon la revendication 1, avec 3–50 parties en poids d'un composé de formule II selon la revendication 1 et l'on met le mélange ainsi obtenu sous forme d'une préparation galénique.

**Claims**

1. A drug which contains a compound of the formula I

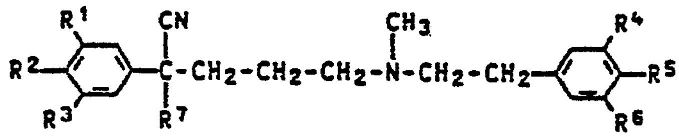

I

where $R^1$, $R^2$, $R^3$, $R^4$, $R^5$ and $R^6$ are identical or different and are each hydrogen, halogen, $C_1$–$C_4$-alkyl or $C_1$–$C_4$-alkoxy, and $R^7$ is a saturated or unsaturated hydrocarbon radical of not more than 20 carbon atoms, and a pyridazinone derivative of the formula II

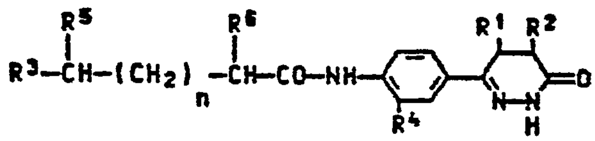

II

where $R^1$ is hydrogen or methyl, $R^2$ is hydrogen or, together with $R^1$, forms methylene or ethylene, $R^3$ is
a) a group of the formula

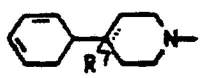

where the dashed line may be an additional bond and, where relevant, $R^7$ is hydrogen or $C_1$–$C_4$-acyl, or
b) a groupe of the formula

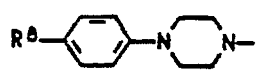

where $R^8$ is hydrogen or $C_1$–$C_4$-acyl, or
c) 1,3-tetrahydroisoquinolin-2-yl,
$R^4$ is hydrogen or, together with $R^3$, forms a direct bond or methylene,
$R^5$ is hydrogen or methyl,
$R^6$ is hydrogen or halogen and
n is 0 or 1,
compound I being present in an amount of 50–450 parts by weight and compound II in an amount of 3–50 parts by weight.

2. A process for the preparation of a drug as claimed in claim 1, wherein 50–450 parts by weight of a compound of the formula I as claimed in claim 1 are mixed with 3–50 parts by weight of a compound of the formula II as claimed in claim 1, and the mixture thus obtained is converted to a pharmaceutical administration form.